# EUROPÄISCHE PATENTSCHRIFT

(11) **EP 4 437 948 B1**
(45) Veröffentlichungstag und Bekanntmachung des Hinweises auf die Patenterteilung: **18.03.2026**
(21) Anmeldenummer: 23165157.1
(22) Anmeldetag: 29.03.2023
(51) Int. Cl.: A61B 5/055

(54) **BILDGEBUNGSVORRICHTUNG, LUFTZUFÜHRUNGSEINRICHTUNG UND VERFAHREN ZUR MONTAGE EINER LUFTZUFÜHRUNGSEINRICHTUNG AN EINER BILDGEBUNGSVORRICHTUNG**
IMAGING DEVICE, AIR SUPPLY DEVICE AND METHOD FOR MOUNTING AN AIR SUPPLY DEVICE ON AN IMAGING DEVICE
DISPOSITIF D'IMAGERIE, DISPOSITIF D'ALIMENTATION EN AIR ET PROCÉDÉ DE MONTAGE D'UN DISPOSITIF D'ALIMENTATION EN AIR SUR UN DISPOSITIF D'IMAGERIE

(43) Veröffentlichungstag der Anmeldung: 02.10.2024
(73) Patentinhaber: Siemens Healthineers AG, 91301 Forchheim (DE)
(72) Erfinder: MACIEJEWSKI, Bernd, 91477 Markt Bibart (DE); SCHRÖDER, Martin, 91096 Möhrendorf (DE)
(74) Vertreter: Siemens Healthineers Patent Attorneys

(56) Entgegenhaltungen:
- EP-B1- 3 501 393
- US-B2- 11 324 417
- US-B2- 8 362 774

## Beschreibung

Die Erfindung betrifft eine Bildgebungsvorrichtung, und eine Luftzuführungseinrichtung für eine Bildgebungsvorrichtung.

Bildgebungsvorrichtungen, wie beispielsweise Magnetresonanztomographen oder Computertomographen weisen eine Aufnahmeröhre auf, in welcher das zu untersuchende Objekt, beispielsweise ein Patient, zur Untersuchung anzuordnen ist. Um eine ausreichende Luftzufuhr sicherzustellen, weisen die Bildgebungsvorrichtungen Luftbereitstellungseinrichtungen auf, die dazu eingerichtet sind, Luft aus einer Umgebung anzusaugen und in die Aufnahmeröhre auszugeben. Dabei wird insbesondere ein laminarer Luftstrom durch die Aufnahmeröhre angestrebt, um eine Wärmeabfuhr aus der Aufnahmeröhre zu ermöglichen.

Da ein Längsabschnitt in einer Mitte der Aufnahmeröhre, wobei es sich um einen Untersuchungsabschnitt handeln kann, durch eine Magneteinrichtung oder eine andere Messvorrichtung umschlossen ist, ist es bautechnisch nicht durchführbar, in diesem Bereich der Bildgebungsvorrichtung Kanäle oder Schläuche der Belüftungseinrichtung anzuordnen, um eine Ausgabe der Luft durch die Belüftungseinrichtung in den Längsabschnitt in der Mitte der Aufnahmeröhre zu ermöglichen. Die Luftbereitstellung erfolgt aus diesem Grund in einen, an eine hintere Öffnung der Aufnahmeröhre angrenzenden Randabschnitt, welcher auch als Rearfunnelabschnitt bezeichnet wird.

Auch wenn die Ausgabe der Luft in Richtung der Mitte der Aufnahmeröhre entlang einer Längsrichtung der Aufnahmeröhre erfolgt, ergibt sich die Problematik, dass ein Hauptluftstrom durch die Aufnahmeröhre für eine Abführung von Wärme aus dem Untersuchungsabschnitt unzureichend sein kann. Diese Problematik ist besonders bei Untersuchungen eines Abschnittes des Objektes, beispielsweise eines Kopfes eines Patienten, mittels Lokalspulen relevant. Die Aufnahmeröhren hierfür ausgelegter Bildgebungsvorrichtungen weisen oft einen geringeren Durchmesser auf. Eine durchströmbare Querschnittsfläche der Aufnahmeröhren wird zudem durch die in der Aufnahmeröhre in dem Untersuchungsabschnitt angeordnete Lokalspuleneinrichtung, beispielsweise einer Kopfspule welche um einen Kopf eines Patienten angeordnet ist, weiter verringert.

Es ist eine Aufgabe der Erfindung, eine Luftzuführung in eine Aufnahmeröhre einer Bildgebungsvorrichtung mit einer darin angeordneten Lokalspuleneinrichtung zu optimieren.

Diese Aufgabe wird gelöst durch den jeweiligen Gegenstand der unabhängigen Ansprüche. Vorteilhafte Weiterbildungen und bevorzugte Ausführungsformen sind Gegenstand der abhängigen Ansprüche.

Ein erster Aspekt der Erfindung betrifft eine Bildgebungsvorrichtung. Bei der Bildgebungsvorrichtung kann es sich insbesondere um einen Magnetresonanztomographen handeln. Die Bildgebungsvorrichtung weist eine Aufnahmeröhre auf. Die Aufnahmeröhre kann zumindest abschnittsweise von einer Magneteinrichtung der Bildgebungsvorrichtung umschlossen sein. Die Aufnahmeröhre kann dazu vorgesehen sein, ein durch die Bildgebungsvorrichtung zu untersuchendes Objekt aufzunehmen.

Die Aufnahmeröhre weist an einem vorderen Längsende eine Aufnahmeöffnung zum Einführen des durch die Bildgebungsvorrichtung zu untersuchenden Objektes auf. Mit anderen Worten ist die Aufnahmeröhre durch die Bildgebungsvorrichtung umschlossen, wobei die Aufnahmeröhre an dem vorderen Längsende die Aufnahmeöffnung aufweist. Die Aufnahmeöffnung kann beispielsweise zum Einführen einer Liegeeinrichtung oder einer Trageeinrichtung zur Anordnung des zu untersuchenden Objektes in die Aufnahmeröhre vorgesehen sein.

Die Aufnahmeröhre weist an einem, dem vorderen Längsende entgegengesetztem hinteren Längsende eine hintere Öffnung auf. Mit anderen Worten ist es vorgesehen, dass die Aufnahmeröhre an dem vorderen Längsende die Aufnahmeöffnung aufweist und an dem hinteren Längsende die hintere Öffnung.

Die Bildgebungsvorrichtung weist eine Belüftungseinrichtung auf, die dazu eingerichtet ist, Luft an einer Luftbereitstellungseinrichtung der Belüftungseinrichtung bereitzustellen, wobei die Luftbereitstellungseinrichtung in einem an die hintere Öffnung angrenzenden Rearfunnelabschnitt der Aufnahmeröhre angeordnet ist. Mit anderen Worten ist die Belüftungseinrichtung dazu eingerichtet, die Luft anzusaugen und die Luft an der Luftbereitstellungseinrichtung bereitzustellen. Die Luftbereitstellungseinrichtung kann beispielsweise ein Ende einer Röhre, eines Schlauches oder eine Düse umfassen. Die Luftbereitstellungseinrichtung ist in dem an die hintere Öffnung angrenzenden Rearfunnelabschnitt der Aufnahmeröhre angeordnet. Die Luftbereitstellungseinrichtung kann sich beispielsweise innerhalb eines Rearcovers der Bildgebungsvorrichtung befinden. Die Luftbereitstellungseinrichtung kann eine Öffnung an oder in der Aufnahmeröhre umfassen, welche sich innerhalb des Rearfunnelabschnitts befinden kann.

Die Bildgebungsvorrichtung weist eine Luftzuführungseinrichtung auf, die dazu eingerichtet ist, einen Hauptluftstrom der ausgegebenen Luft von der Luftbereitstellungseinrichtung entlang einer Längsrichtung der Aufnahmeröhre über eine vorgegebene Luftzuführungslänge zu führen und an einer an einem Ende der Luftzuführungslänge bereitgestellten Luftzuführungsöffnung der Luftausgabeeinrichtung in die Aufnahmeröhre auszugeben. Mit anderen Worten ist es vorgesehen, dass die Bildgebungsvorrichtung die Luftzuführungseinrichtung aufweist. Die Luftzuführungseinrichtung ist dazu eingerichtet, die an der Luftbereitstellungseinrichtung bereitgestellte Luft in die Aufnahmeröhre zu führen. Die Luftzuführungseinrichtung kann insbesondere dazu eingerichtet sein, die bereitgestellte Luft in einem Untersuchungsbereich der Aufnahmeröhre bereitzustellen, welcher sich in einer Mitte der Aufnahmeröhre befinden kann. Die Luft kann insbesondere entlang der Längsrichtung der Aufnahmeröhre in Richtung der Aufnahmeöffnung geleitet werden. Die Luftzuführungseinrichtung ist dazu eingerichtet, den Hauptluftstrom der ausgegebenen Luft von der Luftbereitstellungseinrichtung entlang der Längsrichtung der Aufnahmeröhre über die vorgegebene Luftzuführungslänge zu führen und an einer Zuführungsöffnung der Luftzuführungseinrichtung auszugeben. Die Luftzuführungseinrichtung kann beispielsweise eine Kanaleinrichtung aufweisen, durch welche ein Kanal oder mehrere der Kanäle bereitgestellt sein können, welche entlang der Längsrichtung der Aufnahmeröhre verlaufen und die Luft in einen Abschnitt in eine Mitte der Aufnahmeröhre führen können. Die Luftzuführungslänge kann bevorzugt derart bemessen sein, dass die Luft in einen Untersuchungsabschnitt der Aufnahmeröhre ausgegeben wird. Der Untersuchungsabschnitt kann beispielsweise einen vorgegebenen Aufenthaltsabschnitt beschreiben, in welchem sich während der Untersuchung des Objektes durch die Bildgebungsvorrichtung die Lokalspuleneinrichtung befindet.

Durch die Erfindung ergibt sich der Vorteil, dass eine ausreichende Luftzuführung in räumlich beschränkten Aufnahmeröhren der Bildgebungsvorrichtung bereitgestellt werden kann.

Eine Weiterbildung der Erfindung sieht vor, dass die Zuführungsöffnung in oder an einem Untersuchungsabschnitt der Aufnahmeröhre angeordnet ist. Mit anderen Worten ist es vorgesehen, dass sich die Zuführungsöffnung der Luftzuführungseinrichtung innerhalb des Untersuchungsabschnitts der Aufnahmeröhre befindet oder dass sich die Zuführungsöffnung der Luftzuführungseinrichtung an einem Ende des Untersuchungsabschnitts in der Aufnahmeröhre befindet. Der Untersuchungsabschnitt der Aufnahmeröhre kann beispielsweise ein entlang der Längsrichtung der Aufnahmeröhre angeordneter Abschnitt der Aufnahmeröhre sein, der sich in einem mittleren Abschnitt der Aufnahmeröhre befindet, und in welchem das zu untersuchende Objekt und/oder eine Lokalspuleneinrichtung zur Untersuchung des Objektes zur Untersuchung des Objektes durch die Bildgebungsvorrichtung angeordnet ist. Es kann sich beispielsweise um einen Bereich handeln in dem sich die Lokalspuleneinrichtung bei einer eingefahrenen Liegeeinrichtung der Bildgebungsvorrichtung in der Aufnahmeröhre befinden kann. Durch die Weiterbildung ergibt sich der Vorteil, dass die Luft in dem betroffenen Untersuchungsbereichs bereitgestellt werden kann.

Eine Weiterbildung der Erfindung sieht vor, dass die Luftzuführungseinrichtung stoffschlüssig mit einer Innenwand der Aufnahmeröhre verbunden ist. Mit anderen Worten ist die Luftzuführungseinrichtung stoffschlüssig an der Innenwand angeordnet. Die Aufnahmeröhre kann beispielsweise eine Innenwand aufweisen in welche entlang der Längsrichtung verlaufende Kanäle bereitgestellt sein können. Durch die Weiterbildung ergibt sich der Vorteil, dass aufgrund der formschlüssigen Einarbeitung keine Befestigungsmittel zu Befestigung der Kanäle bereitgestellt werden müssen.

Eine Weiterbildung der Erfindung sieht vor, dass die Luftzuführungslänge eine Länge von mindestens 80 cm aufweist. Mit anderen Worten ist es vorgesehen, dass die Luftzuführungseinrichtung dazu eingerichtet ist, den Hauptluftstrom über eine Länge von mindestens 80 cm entlang der Längsrichtung der Aufnahmeröhre in die Aufnahmeröhre hinein zu führen.

Eine Weiterbildung der Erfindung sieht vor, dass die Luftzuführungseinrichtung dazu eingerichtet ist, den Hauptluftstrom an der Zuführungsöffnung in die Längsrichtung der Aufnahmeröhre auszugeben. Mit anderen Worten ist es vorgesehen, dass der an der Aufnahmeröhre ausgegebene Hauptluftstrom entlang der Längsrichtung der Aufnahmeröhre ausgegeben wird. Es kann beispielsweise vorgesehen sein, dass die Zuführungsöffnung derart ausgerichtet ist, dass der Hauptluftstrom entlang der Längsrichtung der Aufnahmeröhre verläuft. Durch die Weiterbildung ergibt sich der Vorteil, dass ein Luftstrom bereitgestellt werden kann, der durch die Aufnahmeröhre hindurch strömt.

Eine Weiterbildung der Erfindung sieht vor, dass die Luftzuführungseinrichtung dazu eingerichtet ist, den Hauptluftstrom in die Längsrichtung mit einer laminaren Hauptströmungscharakteristik auszugeben. Mit anderen Worten wird die Luft durch die Luftzuführungseinrichtung entlang der Längsrichtung ausgegeben, wobei die Luftzuführungseinrichtung derart parameterisiert ist, dass der Hauptluftstrom der ausgegebenen Luft die laminare Hauptströmungscharakteristik aufweist. Durch die Bereitstellung der laminaren Hauptströmungscharakteristik ist es möglich, durch Verwirbelungen entstehende Verschlechterungen der Luftzuführung zu reduzieren.

Erfindungsgemäß weist die Luftkanaleinrichtung eine Anschlusseinrichtung auf, wobei die Anschlusseinrichtung an der Luftbereitstellungseinrichtung angeordnet und dazu eingerichtet ist, die Luft von der Luftbereitstellungseinrichtung in die Kanaleinrichtung zu führen. Die Luftkanaleinrichtung ist dazu eingerichtet, die Luft von der Anschlusseinrichtung zu der Zuführungsöffnung zu führen. Mit anderen Worten umfasst die Luftzuführungseinrichtung zumindest den Luftkanaleinrichtung und die Anschlusseinrichtung. Die Anschlusseinrichtung kann beispielsweise ein Anschlussmodul zur Anordnung an der Luftbereitstellungseinrichtung sein, welches beispielsweise an einem Rohr oder Schlauch der Luftbereitstellungseinrichtung angeordnet werden kann und welche dazu eingerichtet ist, den Hauptstrom von der Luftbereitstellungseinrichtung in die Luftkanaleinrichtung zu führen. Die Anschlusseinrichtung kann beispielsweise einen Winkel aufweisen und dazu eingerichtet sein, den Hauptluftstrom entlang der Kanallängsrichtung in die Luftkanaleinrichtung zu führen. Die Anschlusseinrichtung kann insbesondere strömungstechnisch derart parameterisiert sein, dass Verwirbelungen der Luft bei der Führung des Hauptluftstroms von der Luftzuführungseinrichtung in die Luftkanaleinrichtung reduziert sind. Die Luftkanaleinrichtung kann an der Anschlusseinrichtung angeordnet sein. Die Anordnung kann beispielsweise durch eine Steckverbindung, eine Schraubverbindung oder Klemmverbindung verbunden sein.

Eine Weiterbildung der Erfindung sieht vor, dass die Anschlusseinrichtung lösbar an der Luftbereitstellungseinrichtung angeordnet ist. Mit anderen Worten ist die Luftzuführungseinrichtung derart eingerichtet dass die Anschlusseinrichtung zerstörungsfrei von der Luftbereitstellungseinrichtung entfernt werden kann. Die Anschlusseinrichtung kann beispielsweise Klemmelemente oder Schraubelemente aufweisen um an die Luftbereitstellungseinrichtung angeschlossen zu werden. Durch die Weiterbildung ergibt sich der Vorteil dass die Luftbereitstellungseinrichtung in vorhandene Bildgebungsvorrichtung integrierbar ist und in Abhängigkeit eines Betriebs der Bildgebungsvorrichtung variabel hinzugefügt oder entfernt werden kann.

Eine Weiterbildung der Erfindung sieht vor, dass die Luftkanaleinrichtung lösbar an der Anschlusseinrichtung angeordnet ist. Mit anderen Worten ist die Luftzuführungseinrichtung derart eingerichtet, dass die Luftkanaleinrichtung zerstörungsfrei von der Anschlusseinrichtung entfernt werden kann. Die Luftkanaleinrichtung kann beispielsweise in die Anschlusseinrichtung verschoben, verklemmt oder verschraubt werden, wobei die Luftkanaleinrichtung und die Anschlusseinrichtung mechanisch zueinander korrespondierende Elemente aufweisen können. Durch die Weiterbildung ergibt sich der Vorteil, dass die Luftkanaleinrichtung in Abhängigkeit eines Betriebs getauscht werden kann. Es kann vorgesehen sein, dass die Luftkanaleinrichtung in Abhängigkeit einer verwendeten Lokalspuleneinrichtung oder in Abhängigkeit einer Art einer Anordnung der Lokalspuleneinrichtung ausgetauscht werden kann.

Erfindungsgemäß weist die Luftzuführungseinrichtung einen Längenverstellmechanismus zur Einstellung der Luftzuführungslänge auf. Mit anderen Worten ist die Luftzuführungseinrichtung dazu eingerichtet, eine Änderung der Luftzuführungslänge zu ermöglichen. Es kann, also vorgesehen sein das die Luftkanaleinrichtung einen Teleskopmechanismus aufweist, wodurch die Luftzuführungslänge der Luftkanaleinrichtung durch ein Verschieben eines Elements des Längenverstellmechanismus der Luftkanaleinrichtung verändert werden kann. Durch die Weiterbildung ergibt sich der Vorteil, dass die Luftzuführungslänge ohne einen Austausch der Luftkanaleinrichtung angepasst werden kann.

Eine Weiterbildung der Erfindung sieht vor, dass die Innenwand der Aufnahmeröhre eine Verbindungseinrichtung zur Anordnung der Luftkanaleinrichtung in der Aufnahmeröhre aufweist. Mit anderen Worten weist die Innenwand der Aufnahmeröhre zur Anordnung der Luftkanaleinrichtung an der Innenwand die Verbindungseinrichtung auf. Die Innenwand der Aufnahmeröhre kann beispielsweise zu der Luftkanaleinrichtung korrespondierende formschlüssige Elemente aufweisen, welche die Anordnung der Luftkanaleinrichtung an der Innenwand ermöglichen. Es kann beispielsweise vorgesehen sein, dass die Innenwand Führungsschienen aufweist, die derart parametriert sind, dass sie ein Einschieben der Luftkanaleinrichtung oder Einklemmen der Luftkanaleinrichtung an die Innenwand ermöglichen. Durch die Weiterbildung ergibt sich der Vorteil, dass eine Anordnung der Luftkanaleinrichtung Vibrationen durch die Bereitstellung der Verbindungseinrichtung reduziert werden können.

Eine Weiterbildung der Erfindung sieht vor, dass die Luftzuführungseinrichtung in dem Untersuchungsabschnitt mit einer geringeren Höhe in die Aufnahmeröhre hineinragt, als außerhalb des Untersuchungsabschnitts. Mit anderen Worten weist die Luftzuführungseinrichtung die Höhe auf, mit welcher die Luftzuführungseinrichtung in eine Radialrichtung der Aufnahmeröhre von der Innenwand der Aufnahmeröhre in die Aufnahmeröhre hereinragt. Die Höhe der Luftzuführungseinrichtung innerhalb des Untersuchungsabschnittes weist dabei einen kleineren Wert auf als die Höhe der Luftzuführungseinrichtung außerhalb des Untersuchungsabschnittes. Durch die Weiterbildung ergibt sich der Vorteil, dass die Luftzuführungseinrichtung in den Untersuchungsabschnitt ragen kann, ohne an die Lokalspuleneinrichtung anzustoßen. Es kann beispielsweise vorgesehen sein, dass die Luftkanaleinrichtung außerhalb des Untersuchungsabschnitts und innerhalb des Untersuchungsabschnitts eine identische Querschnittsfläche zum Führen der Luft bereitstellt. Auf dem Weg zum Untersuchungsabschnitt entlang der Längsrichtung kann die Höhe der Luftkanaleinrichtung abnehmen. Gleichzeitig kann eine tangentiale Abmessung der Luftkanaleinrichtung zu nehmen, um die Abnahme der Höhe auszugleichen.

Eine Weiterbildung der Erfindung sieht vor, dass die Luftzuführungseinrichtung zumindest zwei Luftzuführungseinheiten aufweist. Mit anderen Worten weist die Luftzuführungseinrichtung die zumindest zwei Luftzuführungseinheiten auf, die dazu eingerichtet sind, einen jeweiligen Hauptluftstrom durch die Aufnahmeröhre zu führen. Die zumindest zwei Luftzuführungseinheiten können beispielsweise zwei jeweilige Kanäle aufweisen wodurch die Luft entlang von zwei jeweiligen Hauptluftströmen durch die Aufnahmeröhre geführt werden kann. Eine Weiterbildung der Erfindung sieht vor, dass die Bildgebungsvorrichtung eine Liegeeinrichtung aufweist, wobei die Liegeeinrichtung eine Lokalspuleneinrichtung zur Aufnahme zumindest eines Teilvolumens des zu untersuchenden Objektes aufweist. Die Bildgebungsvorrichtung ist dazu eingerichtet, die Liegeeinrichtung derart zu verfahren, dass sich die Lokalspuleneinrichtung in der Aufnahmeröhre in dem Untersuchungsabschnitt befindet. Mit anderen Worten ist es vorgesehen, dass die Bildgebungsvorrichtung die manuell oder elektromotorisch verschiebbare Liegeeinrichtung aufweist auf welcher das Untersuchungsobjekt anzuordnen ist. Die Liegeeinrichtung kann die Lokalspuleneinrichtung aufweisen, wobei die Lokalspuleneinrichtung beispielsweise an der Liegeeinrichtung an einer vorbestimmten Position befestigt sein kann oder an der vorbestimmten Position aufliegen kann. Durch die Weiterbildung ergibt sich der Vorteil, dass eine Anordnung der Lokalspuleneinrichtung in den vorbestimmten Untersuchungsabschnitt durch die Liegeeinrichtung ermöglicht werden kann.

Ein zweiter Aspekt der Erfindung betrifft eine Luftzuführungseinrichtung für eine Aufnahmeröhre einer Bildgebungsvorrichtung.

Die Luftzuführungseinrichtung ist dazu eingerichtet, einen Hauptluftstrom einer durch eine

Luftbereitstellungseinrichtung ausgegebenen Luft von der Luftbereitstellungseinrichtung entlang einer Längsrichtung einer Aufnahmeröhre über eine vorgegebene Luftzuführungslänge zu führen und an einer, einem Längsende der Luftzuführungslänge bereitgestellten Zuführungsöffnung der Luftzuführungseinrichtung in die Aufnahmeröhre auszugeben. Mit anderen Worten ist die Luftzuführungseinrichtung zur Anordnung in der Bildgebungsvorrichtung vorgesehen und dazu eingerichtet, die bei der Luftbereitstellungseinrichtung der Bildgebungsvorrichtung ausgegebene Luft in die Aufnahmeröhre zu führen.

Ein Verfahren zur Montage einer Luftzuführungseinrichtung an eine Bildgebungsvorrichtung wird ebenfalls offenbart.

Ein Schritt des Verfahrens umfasst ein Befestigen einer Anschlusseinrichtung der Luftzuführungseinrichtung an einer Luftausgabeeinrichtung der Bildgebungsvorrichtung.

Ein weiterer Schritt des Verfahrens umfasst eine Anordnung einer Luftkanaleinrichtung an der Verbindungseinrichtung.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Unabhängig vom grammatikalischen Geschlecht eines bestimmten Begriffes sind Personen mit männlicher, weiblicher oder anderer Geschlechteridentität mit umfasst.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren gezeigten Merkmale und Merkmalskombinationen können nicht nur in der jeweils angegebenen Kombination, sondern auch in anderen Kombinationen von der Erfindung umfasst sein. Es können insbesondere auch Ausführungen und Merkmalskombinationen von der Erfindung umfasst sein, die nicht alle Merkmale eines ursprünglich formulierten Anspruchs aufweisen. Es können darüber hinaus Ausführungen und Merkmalskombinationen von der Erfindung umfasst, die über die in den Rückbezügen der Ansprüche dargelegten Merkmalskombinationen hinausgehen oder von diesen abweichen.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer Bildgebungsvorrichtung.;
- FIG 2: eine weitere schematische Darstellung einer Bildgebungsvorrichtung mit einer Luftzuführungseinrichtung;
- FIG 3: eine schematische Darstellung einer Anordnung der Luftzuführungseinrichtung an das Belüftungssystem der Bildgebungsvorrichtung;
- FIG 4: eine schematische Darstellung einer Luftkanaleinrichtung;
- FIG 5: eine schematische Darstellung einer Aufnahmeröhre aufweisend eine Luftkanaleinrichtung;
- FIG 6: eine schematische Darstellung einer Anschlusseinrichtung der Luftzuführungseinrichtung; und
- FIG 7: eine schematische Darstellung eines Ablaufs eines Verfahrens zur Montage einer Luftzuführungseinrichtung an einer Bildgebungsvorrichtung.

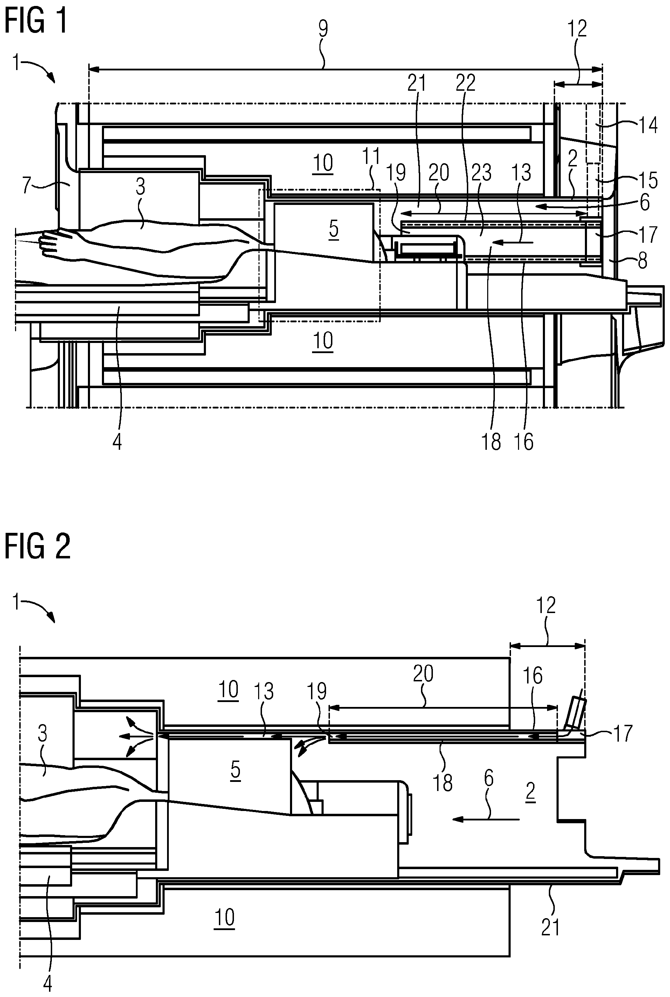

FIG 1 zeigt eine schematische Darstellung einer Bildgebungsvorrichtung.

Bei der gezeigten Bildgebungsvorrichtung 1 kann es sich insbesondere um einen Magnetresonanztomographen handeln. Die Bildgebungsvorrichtung 1 kann eine Aufnahmeröhre 2 aufweisen, welche dazu vorgesehen sein kann, ein durch die Bildgebungsvorrichtung 1 zu untersuchendes Objekt 3 aufzunehmen. Bei dem Objekt 3 kann es sich, beispielsweise um einen Patienten handeln. Das Objekt 3 kann zur Untersuchung durch die Bildgebungsvorrichtung 1 beispielsweise auf einer Liegeeinrichtung 4 der Bildgebungsvorrichtung 1 angeordnet sein, um ein Führen des Objektes 3 in die Aufnahmeröhre 2 zu ermöglichen. Auf der Liegeeinrichtung 4 kann eine Lokalspuleneinrichtung 5 angeordnet sein, wobei es sich beispielsweise um eine Kopfspule handeln kann. Die Lokalspuleneinrichtung 5 kann zur Untersuchung des Objektes 3 in einen vorgegebenen Untersuchungsbereich innerhalb der Aufnahmeröhre 2 geführt werden. Die Aufnahmeröhre 2 kann beispielsweise eine zylindrische Form haben und sich entlang einer Längsrichtung 6 erstrecken. An einem Längsende der Aufnahmeröhre 2 kann die Aufnahmeröhre 2 eine Aufnahmeöffnung 7 aufweisen, in welche das zu untersuchende Objekt 3 eingeführt werden kann. An einem der Aufnahmeöffnung 7 entgegenliegenden Längsende kann die Aufnahmeröhre 2 eine hintere Öffnung 8 aufweisen. Die Aufnahmeröhre 2 kann sich über eine Gesamtlänge 9 erstrecken, wobei die Aufnahmeröhre 2 in einem inneren Bereich der Gesamtlänge 9 durch eine Magneteinrichtung 10 der Bildgebungsvorrichtung 1 umschlossen sein kann. Im Inneren der Aufnahmeröhre 2 kann sich der vorgegebene Untersuchungsbereich befinden. An einem an die hintere Öffnung 8 angrenzenden Randbereich der Aufnahmeröhre 2 kann die Aufnahmeröhre 2 durch einen Rearfunnel oder ein Rearcover umschlossen sein. Zur Wärmeabführung kann es erforderlich sein, Luft durch die Aufnahmeröhre 2 zu leiten. Zu diesem Zweck kann die Bildgebungsvorrichtung 1 eine Belüftungseinrichtung 14 aufweisen, die dazu eingerichtet sein kann, Luft an einer Luftbereitstellungseinrichtung 15 der Belüftungseinrichtung 14 bereitzustellen. Die Luft kann durch die Belüftungseinrichtung 14 beispielsweise innerhalb des Rearcovers oder innerhalb des Rearfunnels bereitgestellt werden.

Nach dem Stand der Technik kann es üblich sein, die Luft innerhalb des Randabschnitts 12, welcher ein Rearfunnelabschnitt sein kann, in die Aufnahmeröhre 2 einzuleiten um die Aufnahmeröhre 2 zu belüften. Dabei ergibt sich jedoch die Problematik, dass ein bereitgestellter Luftstrom durch die Aufnahmeröhre 2 zu gering sein kann, um die Belüftung in einem ausreichenden Maß sicherzustellen.

Die Bildgebungsvorrichtung 1 kann eine Luftzuführungseinrichtung 16 aufweisen, die dazu eingerichtet sein kann, die Luft in einem Inneren der Aufnahmeröhre 2 bereitzustellen. Die Luftzuführungseinrichtung 16 kann beispielsweise eine Luftanschlusseinrichtung 17 aufweisen, die zur Anordnung an der Luftbereitstellungseinrichtung 15 vorgesehen sein kann. Die Anschlusseinrichtung 17 kann beispielsweise lösbar gestaltet sein und beispielsweise an einem Randbereich der Aufnahmeröhre 2 anordenbar sein. Die Luftzuführungseinrichtung 16 kann eine Luftkanaleinrichtung 18 aufweisen, welche dazu eingerichtet sein kann die an der Anschlusseinrichtung 17 bereitgestellte Luft entlang der Längsrichtung 6 der Aufnahmeröhre 2 über eine Zuführungslänge 20 an einer Zuführungsöffnung 19 auszugeben. Die Luftzuführungseinrichtung 16 kann lösbar gestaltet sein und beispielsweise formschlüssig mit einer Verbindungseinrichtung 22 der Innenwand 21 der Aufnahmeröhre 2 verbunden sein. Die Luftkanaleinrichtung 18 kann formschlüssig mit der Innenwand 21 der Aufnahmeröhre 2 verbunden sein. Die Luftzuführungseinrichtung 16 kann einen Verstellmechanismus 23 aufweisen, durch den es ermöglicht sein kann, die Zuführungslänge 20 einzustellen.

FIG 2 zeigt eine weitere Darstellung einer Bildgebungsvorrichtung mit einer Luftzuführungseinrichtung. Die gezeigte Luftzuführungseinrichtung 16 kann die Luftkanaleinrichtung 18 und die Anschlusseinrichtung 17 aufweisen, wobei sowohl die Anschlusseinrichtung 17 als auch die Luftkanaleinrichtung 18 lösbar an einer Innenwand 21 der Aufnahmeröhre 2 angeordnet sein können. Die Anordnung kann beispielsweise mittels eines Stecksystems erfolgen. Die Luftkanaleinrichtung 18 kann auch ein Teleskopsystem aufweisen, wodurch die Zuführungslänge 20 einstellbar sein kann. Die Luftzuführungsöffnung 19 kann derart angeordnet sein, dass sie einen Hauptluftstrom 13 der Luft in den Untersuchungsbereich ausgeben kann. Die Ausgabe kann dabei derart erfolgen, dass die Hauptströmung entlang der Längsrichtung 6 der Aufnahmeröhre 2 ausgegeben wird. Die Luftkanaleinrichtung 18 kann derart parameterisiert sein, dass der Hauptluftstrom 13 eine laminare Hauptstromcharakteristik aufweist kann. Somit können Luftverwirbelungen reduziert werden.

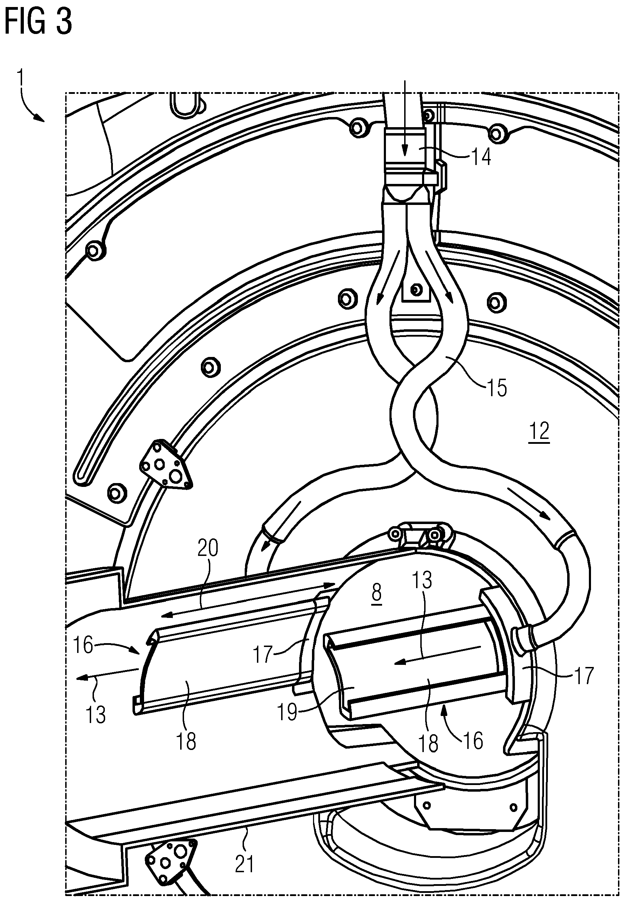

FIG 3 zeigt eine schematische Darstellung einer Anordnung der Luftzuführungseinrichtung an das Belüftungssystem der Bildgebungsvorrichtung.

Gezeigt sind Schläuche und/oder Röhren des Belüftungssystems entlang derer die Luft geführt werden kann. Die Anschlusseinrichtung 17 kann beispielsweise zur Anordnung an eine der Röhren oder einen der Schläuche der Belüftungseinrichtung 14 vorgesehen sein. Die Anschlusseinrichtung 17 kann auch einen Schlauch oder Röhrenabschnitt zur Anordnung an das Belüftungssystem umfassen. Die Schläuche können beispielsweise durch das Rearcover oder den Rearfunnel geführt werden. Die Anschlusseinrichtung 17 kann beispielsweise in eine korrespondierende Form der Innenwand 21 der Aufnahmeröhre 2 gesteckt, geschoben oder verschraubt sein. Die Luftzuführungseinrichtung 16 kann beispielsweise in einen Verbindungsmechanismus der Aufnahmeröhre 2 geklemmt oder geschoben sein und lösbar in die Anschlusseinrichtung 17 gesteckt oder geschoben sein. Die Anschlusseinrichtung 17 kann beispielsweise zwei Anschlusselemente aufweisen, an welchen jeweilige Luftkanalelemente der Luftkanaleinrichtung 18 verbunden sein können. Dadurch können beispielsweise zwei jeweilige der Hauptluftströme 13 durch die jeweiligen Luftkanalelemente geleitet werden.

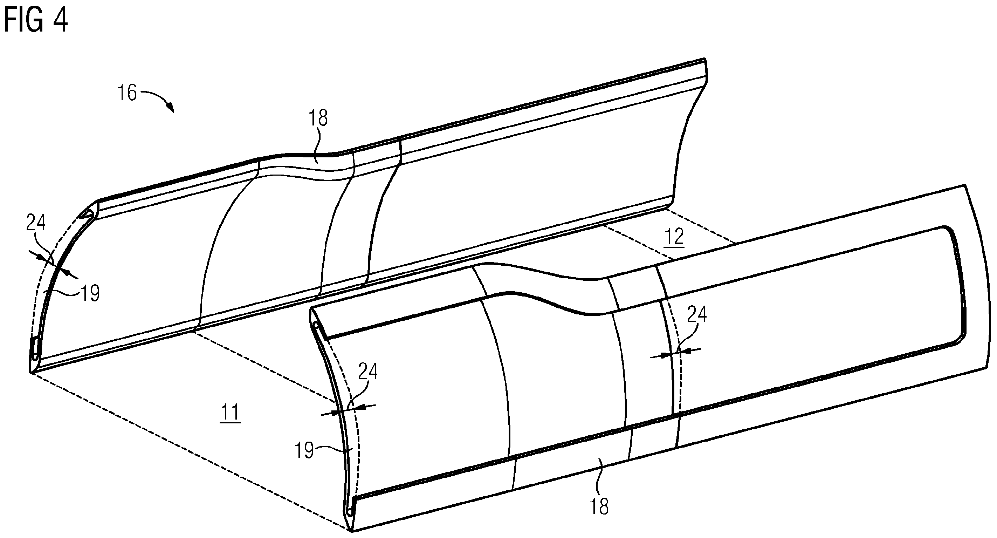

FIG 4 zeigt eine schematische Darstellung einer Luftkanaleinrichtung.

Die Luftkanaleinrichtung 18 kann beispielsweise zur Anordnung an eine Innenwand 21 der Aufnahmeröhre 2 vorgesehen sein. Der Querschnitt der Luftkanaleinrichtung 18 kann sich entlang der Längsrichtung 6 verändern, wobei eine Höhe 24 innerhalb des Untersuchungsabschnitts 11 einen geringeren Wert aufweisen kann als in dem Randabschnitt 12.

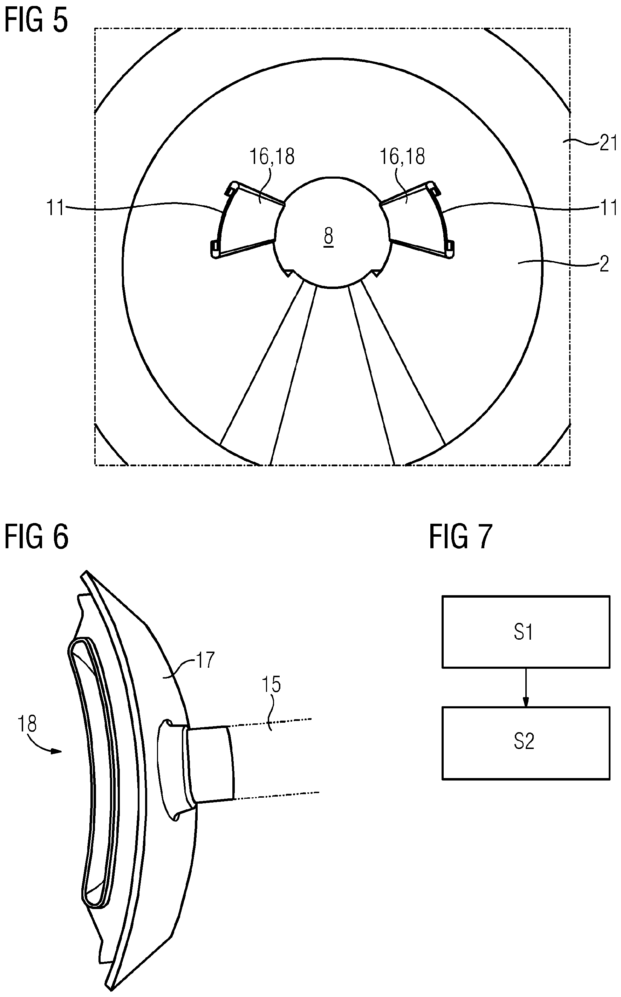

FIG 5 zeigt eine schematische Darstellung einer Aufnahmeröhre aufweisend eine Luftkanaleinrichtung.

Die Luftkanaleinrichtung 18 kann beispielsweise an eine Innenwand 21 der Aufnahmeröhre 2 geklebt sein. Die Öffnung der Luftkanaleinrichtung 18 kann derart ausgerichtet sein, dass die Luft mit einer Hauptströmungsrichtung entlang der Längsrichtung 6 der Aufnahmeröhre 2 ausgegeben werden kann.

FIG 6 zeigt eine schematische Darstellung einer Anschlusseinrichtung der Luftzuführungseinrichtung.

Die Luftzuführungseinrichtung 16 kann die Anschlusseinrichtung 17 aufweisen, welche eine fluidische Verbindung der Luftkanaleinrichtung 18 zu dem Belüftungssystem bereitstellen kann.

FIG 7 zeigt eine schematische Darstellung eines Ablaufs eines Verfahrens zur Montage einer Luftzuführungseinrichtung an einer Bildgebungsvorrichtung.

In einem ersten Schritt S1 des Verfahrens kann es vorgesehen sein, dass eine Anschlusseinrichtung 17 der Luftzuführungseinrichtung 16 an einer Luftbereitstellungseinrichtung 15 einer Belüftungseinrichtung 14 der Bildgebungsvorrichtung 1 angeschlossen wird.

In einem zweiten Schritt S2 des Verfahrens kann es vorgesehen sein, dass eine Luftkanaleinrichtung 18 der Luftzuführungseinrichtung 16 an die Anschlusseinrichtung 17 angeordnet wird.

Bei Bildgebungsvorrichtungen 1, die primär zum Kopf-Scan verwendet werden ist ein Bore-Innendurchmesser der Aufnahmeröhre 2 kleiner als bei gewöhnlichen Bildgebungsvorrichtungen 1 in höheren Stückzahlen. Der Bore-Durchmesser kann beispielsweise lediglich 40 cm im Kopfbereich betragen und führt zu einer schlechteren Patienten-Belüftung. Hinzu kommt, dass die Kopfspulen einen möglichst großen radialen Raum einnehmen und dadurch der Wärme-Abtransport aus dem Bore nochmals deutlich verschlechtert wird.

Herkömmliche Lüftungsdüsen für die Patientenbelüftung sitzen im Rearfunnel. Dadurch ist der Weg bis zur Kopfspule relativ weit und die Luftströmung bei Anwendung im Kopfscanner würde im Bereich zwischen der im Isocenter sitzenden Kopfspule und der hinteren Öffnung 8 im Rearcover/Rearfunnel mehr oder weniger verpuffen, anstatt laminar durch das Bore hindurch zu strömen.

Der kleine Innendurchmesser der Aufnahmeröhre 2 im Bereich des Scans. Der Raum und damit ein Strömungs-Blocker, den die Kopfspule einnimmt. Der lange Weg zwischen Rearcover/Rearfunnel und dem Isocenter. Dieser kann mehr als 100 cm lang sein, da das Rearcover wegen der besonderen Gradientenspule deutlich weiter weg vom Magneten ist.

Die neuen Luftführungen, die die Luft in dem Bereich bereitstellen, der möglichst nahe zu den verschiedenen Kopfspule-Varianten ist. Dabei kann sich die Länge des Lüftungskanals nach der in Z-Richtung entlang einer Längsrichtung längsten Kopfspulen-Type bestimmen.

Es sind auch steckbare Lösungen mit unterschiedlichen Kanal-Längen oder zuschneidbare Kanal-Längen möglich. Dabei wird insbesondere die Servicebarkeit der Luftführungen berücksichtigt, die im Baukasten-Stecksystem ausgeführt sein können.

Die wesentlichen Unterschiede zu bisherigen Patientenbelüftungs-Systemen bestehen hierin:
Die langen Luftführungen ermöglichen eine laminare Luftströmung durch das Bore hindurch, trotz einer voluminösen Kopfspule. Die Strömungsverluste im Bore werden so gering wie möglich gehalten kein Ausblasen in einen großen Hohlraum. Stecksystem der einzelnen Luftführungs-Elemente um die Servicebarkeit des Tragrohres zu gewährleisten. Dadurch, dass die Düsen nicht mehr im Rearfunnel sind, kommt dem eine besondere Beachtung zu. Das Tragrohr muss nach vorne - Patient end - aus dem System ausgebaut werden, d.h. es ist eine vom Tragrohr abnehmbare Luftführungs-Komponente installiert.

## Patentansprüche

1. Bildgebungsvorrichtung (1), aufweisend
- eine Aufnahmeröhre (2), wobei die Aufnahmeröhre (2) an einem vorderen Längsende eine Aufnahmeöffnung (7) zum Einführen eines durch die Bildgebungsvorrichtung (1) zu untersuchenden Objektes (3) aufweist, und an einem dem vorderen Längsende entgegengesetztem hinteren Längsende eine hintere Öffnung (8) aufweist, und
- eine Belüftungseinrichtung (14), die dazu eingerichtet ist, Luft an einer Luftbereitstellungseinrichtung (15) der Belüftungseinrichtung (14) bereitzustellen, wobei die Luftbereitstellungseinrichtung (15) in einem, an die hintere Öffnung (8) angrenzenden Randabschnitt (12) der Aufnahmeröhre (2) angeordnet ist, wobei die Bildgebungsvorrichtung (1) eine Luftzuführungseinrichtung (16) aufweist, wobei die Luftzuführungseinrichtung (16) dazu eingerichtet ist, einen Hauptluftstrom (13) der ausgegebenen Luft von der Luftbereitstellungseinrichtung (15) in einer Längsrichtung (6) der Aufnahmeröhre (2) über eine vorgegebene Luftzuführungslänge (20) zu führen und an einer an einem Längsende der Luftzuführungslänge (20) bereitgestellten Zuführungsöffnung (19) der Luftausgabeeinrichtung in die Aufnahmeröhre (2) auszugeben, wobei die Luftzuführungseinrichtung (16) eine Luftkanaleinrichtung (18) und eine Anschlusseinrichtung (17) aufweist, wobei die Anschlusseinrichtung (17) an der Luftbereitstellungseinrichtung (15) angeordnet ist, und dazu eingerichtet ist, die Luft von der Luftbereitstellungseinrichtung (15) in die Luftkanaleinrichtung (18) zu führen, und die Luftkanaleinrichtung (18) dazu eingerichtet ist, die Luft von der Anschlusseinrichtung (17) zu der Zuführungsöffnung (19) zu führen, **dadurch gekennzeichnet, dass** die Luftzuführungseinrichtung (16) einen Verstellmechanismus (23) zur Einstellung der Luftzuführungslänge (20) aufweist.

2. Bildgebungsvorrichtung (1) nach Anspruch 1 **dadurch gekennzeichnet, dass** die Zuführungsöffnung (19) in oder an einem Untersuchungsabschnitt (11) der Aufnahmeröhre (2) angeordnet ist.

3. Bildgebungsvorrichtung (1) nach einem der vorhergehenden Ansprüche, **dadurch gekennzeichnet, dass** die Luftzuführungslänge (20) eine Länge von mindestens 80 cm aufweist.

4. Bildgebungsvorrichtung (1) nach einem der vorhergehenden Ansprüche, **dadurch gekennzeichnet, dass** die Luftzuführungseinrichtung (16) dazu eingerichtet ist, den Hauptluftstrom (13) in die Längsrichtung (6) der Aufnahmeröhre (2) an der Zuführungsöffnung (19) auszugeben.

5. Bildgebungsvorrichtung (1) nach einem der vorhergehenden Ansprüche, **dadurch gekennzeichnet, dass** die Luftzuführungseinrichtung (16) dazu eingerichtet ist, den Hauptluftstrom (13) in die Längsrichtung (6) mit einer laminaren Hauptströmungscharakteristik auszugeben.

6. Bildgebungsvorrichtung (1) nach einem der vorhergehenden Ansprüche, **dadurch gekennzeichnet, dass** die Anschlusseinrichtung (17) lösbar an der Luftbereitstellungseinrichtung (15) angeordnet ist.

7. Bildgebungsvorrichtung (1) nach einem der vorhergehenden Ansprüche, **dadurch gekennzeichnet, dass** die Luftkanaleinrichtung (18) lösbar an der Anschlusseinrichtung (17) angeordnet ist.

8. Bildgebungsvorrichtung (1) nach einem der vorhergehenden Ansprüche, **dadurch gekennzeichnet, dass** die Innenwand (21) der Aufnahmeröhre (2) eine Verbindungseinrichtung (22) zur Anordnung der Luftkanaleinrichtung (18) in der Aufnahmeröhre (2) aufweist.

9. Bildgebungsvorrichtung (1) nach einem der Ansprüche 1 bis 5, **dadurch gekennzeichnet, dass** die Luftzuführungseinrichtung (16) stoffschlüssig mit einer Innenwand (21) der Aufnahmeröhre (2) verbunden ist.

10. Bildgebungsvorrichtung (1) nach einem der vorhergehenden Ansprüche, **dadurch gekennzeichnet, dass** die Luftzuführungseinrichtung (16) in dem Untersuchungsabschnitt (11) mit einer geringeren Höhe (24) in die Aufnahmeröhre (2) hineinragt, als außerhalb des Untersuchungsabschnitts (11).

11. Bildgebungsvorrichtung (1) nach einem der vorhergehenden Ansprüche, **dadurch gekennzeichnet, dass** die Luftzuführungseinrichtung (16) zumindest zwei Luftzuführungseinheiten aufweist.

12. Bildgebungsvorrichtung (1) nach einem der vorhergehenden Ansprüche, **dadurch gekennzeichnet, dass** die Bildgebungsvorrichtung (1) eine Liegeeinrichtung (4) aufweist, wobei die Liegeeinrichtung (4) eine Lokalspuleneinrichtung (5) zur Aufnahme zumindest eines Teilvolumens des zu untersuchenden Objektes (3) aufweist, wobei die Bildgebungsvorrichtung (1) dazu eingerichtet ist, die Liegeeinrichtung (4) derart zu verfahren, dass sich die Lokalspuleneinrichtung (5) in dem Untersuchungsabschnitt (11) befindet

13. Luftzuführungseinrichtung (16) für eine Aufnahmeröhre einer Bildgebungsvorrichtung (1), wobei die Luftzuführungseinrichtung (16) dazu eingerichtet ist, einen Hauptluftstrom (13) einer durch eine Luftbereitstellungseinrichtung (15) ausgegebenen Luft von der Luftbereitstellungseinrichtung (15) in einer Längsrichtung (6) einer Aufnahmeröhre (2) über eine vorgegebene Luftzuführungslänge (20) zu führen und an einer an einem Längsende der Luftzuführungslänge (20) bereitgestellten Zuführungsöffnung (19) der Luftausgabeeinrichtung in die Aufnahmeröhre (2) auszugeben, wobei die Luftzuführungseinrichtung (16) eine Luftkanaleinrichtung (18) und eine Anschlusseinrichtung (17) aufweist, wobei die Anschlusseinrichtung (17) an der Luftbereitstellungseinrichtung (15) angeordnet ist, und dazu eingerichtet ist, die Luft von der Luftbereitstellungseinrichtung (15) in die Luftkanaleinrichtung (18) zu führen, und die Luftkanaleinrichtung (18) dazu eingerichtet ist, die Luft von der Anschlusseinrichtung (17) zu der Zuführungsöffnung (19) zu führen,
**dadurch gekennzeichnet, dass** die Luftzuführungseinrichtung (16) einen Verstellmechanismus (23) zur Einstellung der Luftzuführungslänge (20) aufweist.

## Claims

1. Imaging apparatus (1), having
- a receive tube (2), wherein
the receive tube (2) has a receive aperture (7) at a front longitudinal end for inserting an object to be examined (3) by the imaging apparatus (1), and has a rear aperture (8) at a rear longitudinal end opposite the front longitudinal end, and
- a ventilation facility (14) which is configured to provide air to an air provision facility (15) of the ventilation facility (14), wherein the air provision facility (15) is arranged in an edge portion (12) of the receive tube (2) adjacent to the rear aperture (8),
wherein the imaging apparatus (1) has an air supply facility (16), wherein the air supply facility (16) is configured to guide a main air flow (13) of the air which is output by the air provision facility (15) in a longitudinal direction (6) of the receive tube (2) over a prespecified air supply length (20) and to output the main air flow into the receive tube (2) at a supply aperture (19) of the air output facility provided at a longitudinal end of the air supply length (20),
wherein the air supply facility (16) has an air channel facility (18) and a connection facility (17), wherein the connection facility (17) is arranged on the air provision facility (15), and is configured to guide the air from the air provision facility (15) into the air channel facility (18), and the air channel facility (18) is configured to guide the air from the connection facility (17) to the supply aperture (19),
**characterised in that** the air supply facility (16) has an adjustment mechanism (23) for setting the air supply length (20).

2. Imaging apparatus (1) according to claim 1, **characterised in that** the supply aperture (19) is arranged in or on an examination section (11) of the receive tube (2).

3. Imaging apparatus (1) according to one of the preceding claims, **characterised in that** the air supply length (20) has a length of at least 80 cm.

4. Imaging apparatus (1) according to one of the preceding claims, **characterised in that** the air supply facility (16) is configured to output the main air flow (13) at the supply aperture (19) in the longitudinal direction (6) of the receive tube (2).

5. Imaging apparatus (1) according to one of the preceding claims, **characterised in that** the air supply facility (16) is configured to output the main air flow (13) in the longitudinal direction (6) with a main flow characteristic that is laminar.

6. Imaging apparatus (1) according to one of the preceding claims, **characterised in that** the connection facility (17) is detachably arranged on the air provision facility (15).

7. Imaging apparatus (1) according to one of the preceding claims, **characterised in that** the air channel facility (18) is detachably arranged on the connection facility (17).

8. Imaging apparatus (1) according to one of the preceding claims, **characterised in that** the inner wall (21) of the receive tube (2) has a connection facility (22) for arranging the air channel facility (18) in the receive tube (2).

9. Imaging apparatus (1) according to one of claims 1 to 5, **characterised in that** the air supply facility (16) is connected with a material bond to an inner wall (21) of the receive tube (2).

10. Imaging apparatus (1) according to one of the preceding claims, **characterised in that** the air supply facility (16) in the examination section (11) projects into the receive tube (2) at a lower height (24) than outside the examination section (11).

11. Imaging apparatus (1) according to one of the preceding claims, **characterised in that** the air supply facility (16) has at least two air supply units.

12. Imaging apparatus (1) according to one of the preceding claims, **characterised in that** the imaging apparatus (1) has a couch facility (4), wherein the couch facility (4) has a local coil facility (5) for receiving at least a partial volume of the object to be examined (3), wherein the imaging apparatus (1) is configured to move the couch facility (4) such that the local coil arrangement (5) is situated in the examination section (11).

13. Air supply facility (16) for a receive tube of an imaging apparatus (1), wherein the air supply facility (16) is configured to guide a main air flow (13) of air which is output by an air provision facility (15) from the air provision facility (15) in a longitudinal direction (6) of a receive tube (2) over a prespecified air supply length (20) and to output the main air flow into the receive tube (2) at a supply aperture (19) of the air output facility provided at a longitudinal end of the air supply length (20),
wherein the air supply facility (16) has an air channel facility (18) and a connection facility (17), wherein the connection facility (17) is arranged on the air provision facility (15), and is configured to guide the air from the air provision facility (15) into the air channel facility (18), and the air channel facility (18) is configured to guide the air from the connection facility (17) to the supply aperture (19),
**characterised in that** the air supply facility (16) has an adjustment mechanism (23) for setting the air supply length (20).

## Revendications

1. Installation (1) d'imagerie comportant
- un tube (2) d'enregistrement, dans laquelle
le tube (2) d'enregistrement a une extrémité longitudinale avant une ouverture (7) de réception pour l'introduction d'un objet (3) à examiner par l'installation (1) d'imagerie et a une ouverture (8) arrière à une extrémité longitudinale arrière opposée à l'extrémité longitudinale avant, et
- un dispositif (14) d'alimentation en air, qui est agencé pour mettre de l'air à disposition d'un dispositif (15) de mise à disposition d'air du dispositif (14) d'alimentation en air, dans laquelle le dispositif (15) de mise à disposition d'air est disposé dans un tronçon (12) marginal voisin de l'ouverture (8) arrière du tube (2) d'enregistrement,
dans laquelle l'installation (1) d'imagerie a un dispositif (16) d'apport d'air, dans laquelle le dispositif (16) d'apport d'air est agencé pour conduire, sur une longueur (20) de conduite d'air donnée à l'avance dans une direction (6) longitudinale du tube (2) d'enregistrement, un courant (13) principal d'air de l'air donné par le dispositif (15) de mise à disposition d'air, et pour l'envoyer dans le tube (2) d'enregistrement à une ouverture du dispositif donnant de l'air, à une ouverture (19) d'apport mise à disposition à une extrémité longitudinale de la longueur (20) d'apport d'air,
dans laquelle le dispositif (16) d'apport d'air a un dispositif (18) à conduit d'air et un dispositif (17) de raccordement, dans laquelle le dispositif (17) de raccordement est monté sur le dispositif (15) de mise à disposition d'air et est agencé pour conduire l'air du dispositif (15) de mise à disposition d'air au dispositif (18) à conduit d'air et le dispositif (18) à conduit d'air est agencé pour conduire l'air du dispositif (17) de raccordement à l'ouverture (19) d'apport,
**caractérisée en ce que** le dispositif (16) d'apport d'air a un mécanisme (23) de réglage pour le réglage de la longueur (20) d'apport d'air.

2. Installation (1) d'imagerie suivant la revendication 1, **caractérisée en ce que** l'ouverture (19) d'apport est disposée dans ou sur un tronçon (11) d'examen du tube (2) d'enregistrement.

3. Installation (1) d'imagerie suivant l'une des revendications précédentes, **caractérisée en ce que** la longueur (20) d'apport d'air a une longueur d'au moins 80 cm.

4. Installation (1) d'imagerie suivant l'une des revendications précédentes, **caractérisée en ce que** le dispositif (16) d'apport d'air est agencé pour donner le courant (13) principal d'air à l'ouverture (19) d'apport dans la direction (6) longitudinale du tube (2) d'enregistrement.

5. Installation (1) d'imagerie suivant l'une des revendications précédentes, **caractérisée en ce que** le dispositif (16) d'apport d'air est agencé pour donner le courant (13) principal d'air dans la direction (6) longitudinale avec une caractéristique d'écoulement principale laminaire.

6. Installation (1) d'imagerie suivant l'une des revendications précédentes, **caractérisée en ce que** le dispositif (17) de raccordement est monté de manière amovible sur le dispositif (15) de mise à disposition d'air.

7. Installation (1) d'imagerie suivant l'une des revendications précédentes, **caractérisée en ce que** le dispositif (18) à conduit d'air est monté de manière amovible sur le dispositif (17) de raccordement.

8. Installation (1) d'imagerie suivant l'une des revendications précédentes, **caractérisée en ce que** la paroi (21) intérieure du tube (2) d'enregistrement a un dispositif (22) de liaison pour le montage du dispositif (18) à conduit d'air dans le tube (2) d'enregistrement.

9. Installation (1) d'imagerie suivant l'une des revendications 1 à 5, **caractérisée en ce que** le dispositif (16) d'apport d'air est relié à coopération de matière à une paroi (21) intérieure du tube (2) d'enregistrement.

10. Installation (1) d'imagerie suivant l'une des revendications précédentes, **caractérisée en ce que** le dispositif (16) d'apport d'air dans le tronçon (11) d'examen pénètre à un niveau (24) plus petit dans le tube (2) d'enregistrement qu'à l'extérieur du tronçon (11) d'examen.

11. Installation (1) d'imagerie suivant l'une des revendications précédentes, **caractérisée en ce que** le dispositif (16) d'apport d'air a au moins deux unités d'apport d'air.

12. Installation (1) d'imagerie suivant l'une des revendications précédentes, **caractérisée en ce que** l'installation (1) d'imagerie a un dispositif (4) de couchette, dans laquelle le dispositif (4) de couchette a un dispositif (5) de bobine locale pour l'enregistrement d'au moins un volume partiel de l'objet (3) à examiner, dans laquelle l'installation (1) d'imagerie est agencée pour déplacer le dispositif (4) de couchette, de manière à ce que le dispositif (5) de bobine locale se trouve dans le tronçon (11) d'examen.

13. Dispositif (16) de conduite d'air pour un tube d'enregistrement d'une installation (1) d'imagerie, dans lequel le dispositif (16) d'apport d'air est agencé pour conduire, sur une longueur (20) d'apport d'air donnée à l'avance, dans une direction (6) longitudinale d'un tube (2) d'enregistrement, un courant (13) principal d'air donné par un dispositif (15) pour mettre de l'air à disposition du dispositif (15) de mise à disposition d'air et pour le donner dans le tube (2) d'enregistrement à une ouverture (19) d'apport, mise à disposition à une extrémité longitudinale de longueur (20) d'apport d'air, du dispositif donnant de l'air,
dans lequel le dispositif (16) d'apport d'air a un dispositif (18) à conduit d'air et un dispositif (17) de raccordement, dans lequel le dispositif (17) de raccordement est monté sur le dispositif (15) pour mettre de l'air à disposition et est agencé pour conduire l'air du dispositif (15) de mise à disposition d'air au dispositif (18) à conduit d'air et le dispositif (18) à conduit d'air est agencé pour conduire l'air du dispositif (17) de raccordement à l'ouverture (19) d'apport,
**caractérisé en ce que** le dispositif (16) d'apport d'air a un mécanisme (23) de réglage pour le réglage de la longueur (20) d'apport d'air.
